# EUROPEAN PATENT APPLICATION

(11) **EP 1 267 166 A1**
(43) Date of publication of application: **18.12.2002**
(21) Application number: 01912255.5
(22) Date of filing: 12.03.2001
(51) Int. Cl.: G01N 33/49

(54) **BLOOD ANALYZING METHOD AND APPARATUS**

(30) Priority: 15.03.2000 JP 2000120189
(71) Applicant: Kikuchi, Jun, Minato-ku, Tokyo 108-0071 (JP); Horiike, Yasuhiro, Houya-shi, Tokyo 202-0021 (JP)
(72) Inventor: KIKUCHI, Jun, Tokyo 108-0071 (JP); HORIIKE, Yasuhiro, Nishitokyo-shi Tokyo 202-0021 (JP); OGAWA, Hiroki, Kohoku-kum, Yokohama-shi Kanagawa 222-00 (JP); TAKAMURA, Yuzuru, Arakawa-ku, Tokyo 116-0003 (JP); OKI, Akio, Mitaka-shi, Tokyo 181 0015 (JP); ADACHI, Sakuichiro, Aso-ku Kawasaki-shi, Kanagawa 215-0013 (JP); ICHIKI, Takanori, Sakado-shi, Saitama 350-0214 (JP); ISHIHARA, Kazuhiko, Kodaira-shi, Tokyo 187-0022 (JP)
(74) Representative: Grünecker, Kinkeldey, Stockmair & Schwanhäusser Anwaltssozietät
(86) International application number: JP0101896
(87) International publication number: WO01069242

(57) **Abstract**

A blood analyzing apparatus is provided, which includes a microcapillary fabricated on a substrate by a micromanufacturing technique and used for sampling, filtering, separating, and moving blood and a detector so as to conduct a process from sampling blood to analyzing it using the substrate. A blood analyzing method is also provided for conducting a process from sampling blood to analyzing it by using the blood analyzing apparatus.

## Description

### Technical Field

The present invention relates to a blood analyzing method and a blood analyzing apparatus for sampling blood, removing red blood cells, white blood cells, lymphoid cells, platelets, a blood coagulation factor and the like from the blood to obtain a serum, and measuring a pH value or concentration of oxygen, carbon dioxide or the like of thus obtained serum. The method and the apparatus are particularly characterized in that functions and structures necessary for the above operations are all integrated in one device.

### Background Art

Electronic apparatus for diagnosing a human health condition or disease includes an automatic blood analyzing apparatus in addition to a clinical thermometer, a blood pressure gauge, an ultrasonic diagnostic equipment, an X-ray CT, an MRI and the like. This apparatus is used for sampling blood of several milliliters, removing red blood cells, white blood cells, lymphoid cells, platelets, and a blood coagulation factor by using a centrifuge to obtain a serum, aliquoting the serum into a number of test tubes, aligning and moving the test tubes, and measuring pH value and concentrations of oxygen and carbon dioxide. It is also used for diagnosing a human body by adding a reagent such as an enzyme into the serum of each test tube, subjecting emission reaction with a substrate in the serum to spectroscopic or absorption spectroscopic analysis, and processing data by a computer.

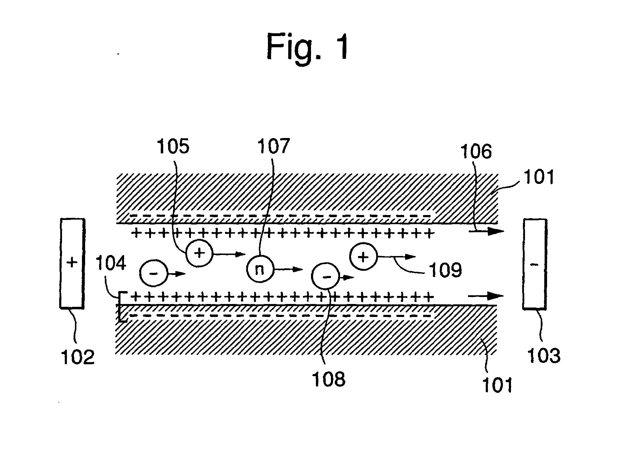

With regard to a technology for a method of analyzing and measuring blood or a human tissue, the following developments have been made. First, as a method of measuring a substance contained in a solution such as serum, capillary electrophoresis is generally used. This method is now described by referring to FIG. 1. A reference numeral 101 denotes a silica tube, 102 a positive electrode, and 103 a negative electrode. For example, an electrolytic solution is put into the elongated silica tube 101 called a capillary having a diameter of about 0.1 mm. Generally, in the case of the silica tube, negative charges are generated on a surface of its inner wall. Thus, cations (ions having positive charges) in the electrolytic solution are concentrated on the inner wall of the silica tube due to a coulomb's force with the negative charges, forming so-called Helmholtz double barrier 104. When high voltages are applied to electrodes 102, 103 provided in both ends of the capillary, first, cations 105 on the inner wall are attracted and moved to the negative voltage 103 side. Then, the entire electrolytic solution is associatively moved to the negative voltage side 103 due to the viscosity thereof. This flow is referred to as an electro osmotic flow 106. On the other hand, the cations in the electrolytic solution reach the negative electrode 103 first, and then neutrals 107 reach it by the electro osmotic flow 106. Anions 108 (negative ions) are ordinarily attracted to the positive voltage side 102. However, the anions 108 are moved to the negative voltage 103 side by the electro osmotic flow 106, thus reaching it last. A flow of such movement of the anions and cations due to electric field is referred to as an electrophoretic flow 109.

With regard to DNA analysis, recently, the following method has been studied. That is, a so-called microcapilliary is available, which is fabricated on a several cm-square chip by forming the capillary on a quartz plate or a polymer plate, and covering it with a capping plate. For example, DNA or the like is put into neutral gel. As it has charges, the DNAs are moved by electrophoresis, and separated based on a total charge difference caused by a difference in molecular weight. This method has been studied to expand into a reaction detecting method different from a conventional test-tube system, in which a reagent is added to a substance moving in the microcapillary in the midway to detect its reaction (e.g., Yoshinobu BABA, "PROTEIN, NUCLEIC ACID, AND ENZYME" Vol. 45, No. 1 (2000) pp.76-85). This is referred to as a total analysis system (µ-TAS) or Lab-on-Chip.

The above-described automatic blood analyzing apparatus is generally expensive and large in size, and therefore is used at a blood center or a hospital. Also total diagnosis requires several hours. Such blood analysis is usually conducted in investigation into a cause of disease. Also the medical examination using blood analysis is usually carried out about once a year even for a healthy body. Thus, it is designed to obtain various bits of detailed information by one round analysis. Recently, however, human health has been daily damaged by the heating of the atmosphere or environmental deterioration by endocrine disrupters. Therefore, it is necessary to frequently conduct blood test for health management to prevent a development to serious disease. In such a blood test for daily health management, it is only necessary to measure concentrations of so-called health markers such as pH, oxygen, carbon dioxide, sodium, potassium, calcium, glucose, and lactic acid or the like. Detailed examination using the above-described blood analyzing apparatus which is high in price and large in size is not necessary, requiring only a simple apparatus for narrowed-down items.

In addition, with a recent increase in the number of bed-ridden aged people, their health care has become a challenge to be addressed. For such people, it is difficult to frequently visit a hospital to take examination. Thus, means must be provided to conduct health management at home. In a current blood testing method, blood sampling is allowed only to a qualified person such as a doctor. A helper responsible for nursing care of the aged who needs care is not allowed to sample blood. Accordingly, a blood analyzing apparatus has been requested, which even an ordinary person can easily handle including blood sampling. To conduct a blood test frequently, no pain is preferably felt in blood sampling. Also, it is preferable to suppress an effect on a human body such as a change of skin color by an injection needle as much as possible.

Therefore, an object of the present invention is to provide a blood analyzing method having no drawbacks, which have been inherent in the blood analyzing method using the conventional blood analyzing apparatus, and easily used at home.

In order to achieve the above-described object, it is another object of the invention to provide a blood analyzing apparatus including functions necessary for blood analysis, such as sampling, filtering, separating and analyzing, integrated in a compact form, and handled by ordinary people without needing any special medical knowledge or qualification for operation.

### Disclosure of the Invention

In accordance with the present invention, there is provided a blood analyzing apparatus which comprises, in one or a plurality of substrates; sampling means for sampling blood from a living body; at least one of filtering means and separating means, the filtering means being for filtering the sampled blood to obtain blood plasma, the separating means being for separating serum from the blood; analyzing means for analyzing a substance in the blood; flow path means for interconnecting the sampling means, the filtering means, the separating means and the analyzing means; moving means for moving components of the blood present in the sampling means, the filtering means, the separating means, the analyzing means and the flow path means; outputting means for taking out information from the analyzing means; and control means for controlling an operation of at least one of the sampling means, the filtering means, the separating means, the analyzing means, the moving means and the outputting means; wherein the substrate further comprises holding means for holding the blood components in the substrate, and if the plurality of the substrates are present, these substrates are integrally constituted. Accordingly, the blood analyzing apparatus is compact, and suitable for installation at ordinary home.

In accordance with the present invention, there is further provided a blood analyzing method which conducts a sequential process of from blood sampling to analyzing by the blood analyzing apparatus. Accordingly, the blood analyzing method is suitable for use by an ordinary person having no special medical knowledge or qualification.

### Brief Description of the Drawings

FIG. 1 is an explanatory view of capillary electrophoresis;
FIG. 2 is a schematic view of an apparatus according to a preferred embodiment of the present invention;
FIG. 3 is an explanatory view of a microcapillary fabrication process;
FIG. 4 is a view showing movement of ions in the microcapillary by an electro osmotic flow;
FIG. 5 is a view showing a method for measuring a suction force of a pumping operation;
FIG. 6 is a view showing a configuration of separating means;
FIG. 7 is a view showing a centrifuge;
FIG. 8 is a view showing an advantage of applying MPC polymer onto an inner wall of the microcapillary;
FIG. 9 is a view showing an effect of a length of the microcapillary on the pumping operation;
FIG. 10 is a view showing a configuration of a chemical sensor;
FIG. 11 is a view showing a result of measuring glucose concentration;
FIG. 12 is a view showing results of measurement of pH value and concentrations of Na⁺ and K⁺;
FIG. 13 is a view showing a configuration of a blood analyzing apparatus provided with an anticoagulant feeder; and
FIG. 14 is a view showing a configuration of a blood analyzing apparatus provided with an anticoagulant feeder.

### Best Mode for Carrying out the Invention

Detailed description will be made of the present invention with reference to the accompanying drawings.

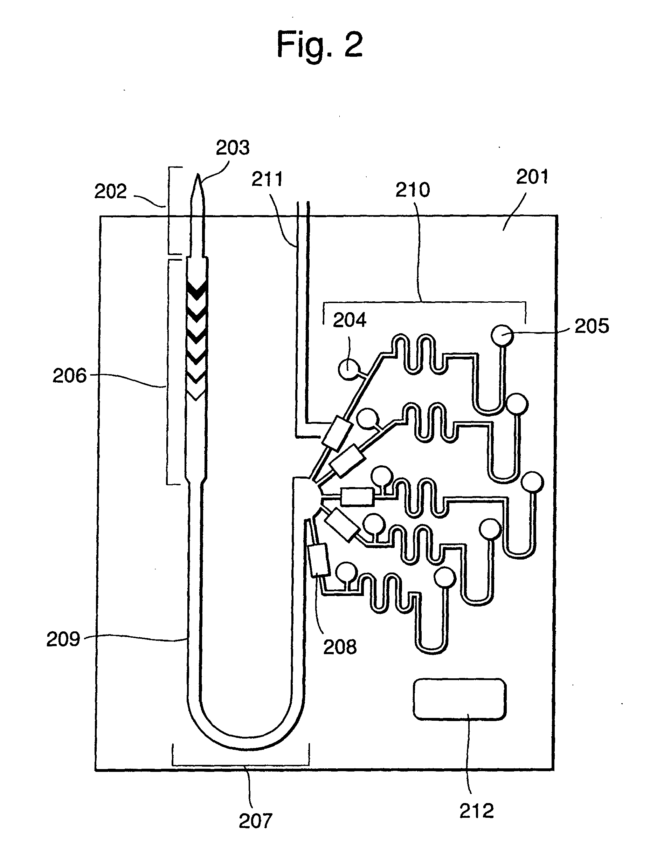

FIG. 2 schematically shows an apparatus according to the invention. A reference numeral 201 denotes a substrate, and each means of the apparatus is arranged along a microcapillary fabricated on the substrate by etching. MPC polymer (2-methacryloyloxyethylphosphorylcholine) is applied onto a surface of the microcapillary, thereby preventing coagulation or adherence of protein in blood plasma or serum on the surface of the microcapillary. A reference numeral 202 denotes blood sampling means, and 203 a hollow needle belonging to the sampling means. This needle is stuck into a body to form an inlet of blood to the substrate. Reference numerals 204, 205 denote electrodes. Blood is taken from within the body into the substrate by a suction force of an electro osmotic flow generated by a voltage applied between the electrodes 204, 205. A reference numeral 206 denotes blood filtering means including a plurality of slits gradually narrowed in space from an upstream side to a downstream side of a blood flow. By these slits, red, white and lymphoid corpuscles and platelets in the blood are filtered to be removed, thereby obtaining blood plasma in the downstream side of the filtering means. A reference numeral 207 denotes separating means including, for example a U-shaped microcapillary. The blood plasma obtained by filtering the sampled blood is introduced to the U-shaped microcapillary. Then, by using a centrifuge to apply acceleration of a given direction to the substrate, a coagulation factor is separated and removed from the blood plasma to obtain a serum in the U-shaped part. A reference numeral 208 denotes analyzing means including a sensor for measuring a pH value, and concentration of each of oxygen, carbon dioxide, sodium, potassium, calcium, glucose, lactic acid and the like in the blood. 209 is flow path or channel means for interconnecting the sampling means, the filtering means, the separating means and the analyzing means, and is formed of a microcapillary fabricated on the substrate by etching. 210 is moving means for moving the blood in the microcapillary by an electro osmotic flow. 211 is outputting means for taking out information from the analyzing means, and includes an electrode or the like. 212 is control means for controlling the sampling means, the filtering means, the separating means, the analyzing means, the moving means and the outputting means when necessary. Though not shown, a plate is provided to hold the blood in the microcapillary on the substrate. This plate is adhered or cramped to the substrate 201.

The blood sampled by the sampling means 202 is filtered by the filtering means 204 to become blood plasma. A coagulation factor is separated and removed by the separating means 205, thereby obtaining serum. Then, a pH value and concentration of each of oxygen, carbon dioxide, sodium, potassium, calcium, glucose, lactic acid and the like in the serum are measured by the analyzing means. Transfer of the blood between the these means is carried out by the moving means 210 using electrophoresis.

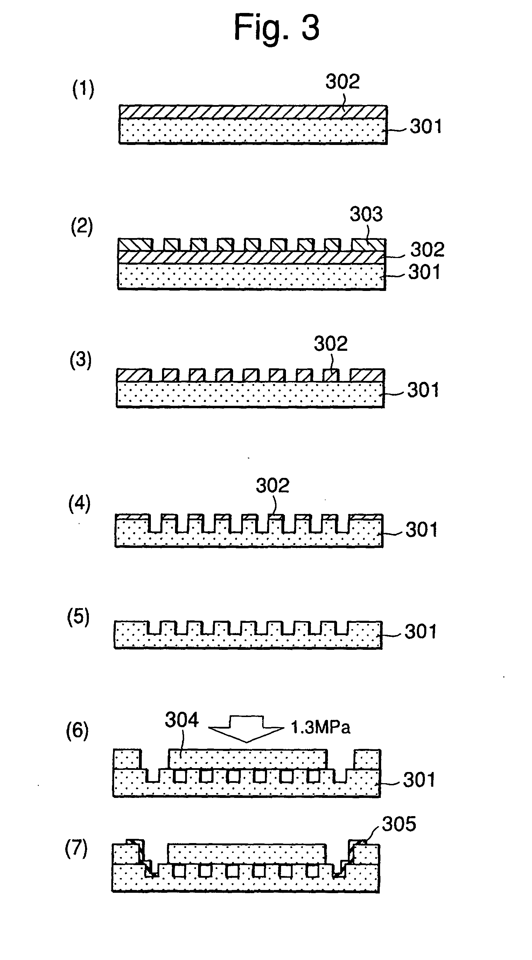

Next, description will be made of a process of fabricating the microcapillary for formation of each means in the substrate by referring to FIG. 3. For example, a 2 cm-square quartz plate 301 having a thickness of 0.5 mm is prepared.
(1) First, a chrome (Cr) is deposited on the quartz plate 301 by a sputtering method to form a Cr film 302 having a thickness of about 1 µm.
(2) Then, a photoresist 303 is applied onto the Cr film 302, and a parting pattern having a width of about 30 µm is formed by exposure.
(3) The Cr film 302 is etched by a wet or dry method. In the dry method, Cl₂ gas is discharged by inductively coupled plasma (ICP) using a coil antenna, and the Cr film 302 is etched in a downstream area of 18 to 20 cm from the antenna. For this etching method, a method disclosed in Japanese Patent Application No. 11-124709 is used.
(4) Then, with the Cr film 302 is used as a mask, and a mixture of C₄F₈ and SF₆ gases at a ratio of 85:15 is discharged by ICP. The quartz plate 301 is placed on the electrode, to which a high-frequency bias of 13.56 MHz has been applied. The quartz plate 301 as a substrate is etched to have a vertical wall, and a capillary groove is formed. Conditions in this case are that the power of 13.56 MHz introduced to the antenna is 500 W, the power of 13.56 MHz for a high-frequency bias is 5 W, and the pressure is 10 mTorr.
(5) Subsequently, the Cr film 302 is removed by the wet method.
(6) Another quartz plate 304 is prepared, which includes an inlet and an outlet for blood or a buffer solution, bored by ultrasonic machining. The quartz plate 304 is dipped in 1% hydrofluoric acid together with the quartz plate 301 having the capillary groove, and then these plates 301, 304 are laminated, and pressed by pressure of 1.3 MPa for 24 hours.
(7) Lastly, an electrode 305 of platinum or the like is formed in the inlet or the outlet by a deposition method or the like, thereby completing a microcapillary chip.

By using the quartz plate having the microcapillary groove formed in the step (4) as a forming die, a mold may be formed on a polymer membrane of polyethylene or the like, thereby forming a microcapillary groove. Further, without using any quartz plates, a microcapillary groove may be directly formed on a polymer plate, and a capping plate may also be made of polymer.

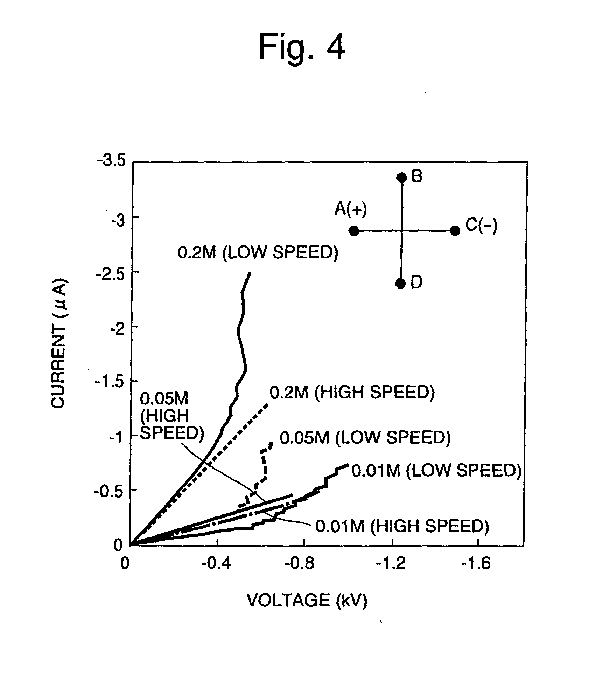

Next, description will be made of a method of drawing blood into the microcapillary by the blood sampling means, and a method of moving blood in the microcapillary by the moving means by referring to FIG. 4. The microcapillary used is shown in a graph of FIG. 4. This is a part of the microcapillary fabricated in the quartz plate by the foregoing process. The microcapillary has a width of about 30 µm, and a depth of about 30 µm. After the microcapillary was filled throughout with a phosphoric buffer solution (PBS) of pH 7.4, a high voltage was applied between A and C, and a voltage-current characteristic at this time was measured by changing ionic strength. A result is shown in the graph of FIG. 4. In the graph, "LOW SPEED" is a result of increasing applied voltage with 50 V for every 15 sec., and "HIGH SPEED" is a result of increasing applied voltage with 70 V per sec. In the case of "LOW SPEED", a current suddenly starts to flow associatively with voltage application, especially steeply when ionic strength is high. This may be attributed to the fact that a current flow causes Joule heating, and mobility of the buffer solution is increased. On the other hand, when a voltage is increased in the case of "HIGH SPEED", a linear relation is exhibited. Accordingly, a sudden voltage increase means high-speed movement of positive ions without any increases in a temperature of the buffer solution. In other words, by using the "HIGH SPEED" mode, an electro osmotic flow serves as a pumping function for moving the solution.

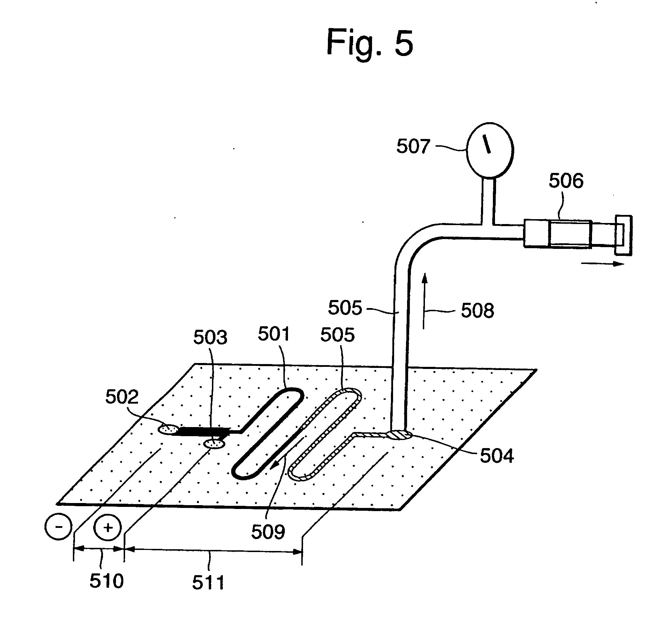

Now, description is made of measurement of a suction force by the pumping function of the electro osmotic flow. FIG. 5 shows a microcapillary having been subjected to measurement of a suction force by a pumping operation. A part cut out from the quartz plate prepared by the foregoing process is shown. The microcapillary has a width of about 30 µm, and a depth of about 30 µm. First, the microcapillary was filled throughout with a phosphoric buffer solution (PBS) of pH 7.4. Then, when high voltages were applied to an electrode A (502) and an electrode B (503), the PBS was moved to the electrode A side by an electro osmotic flow, and air 505 was injected from an inlet C (504). Measurement was made of pressure by a pressure gauge 507 when a fore end of the air 505 having entered the microcapillary was drawn back to the inlet C (504). As a result, a suction force by the pumping operation of the electro osmotic flow was discovered to be 6x10³ Pa.

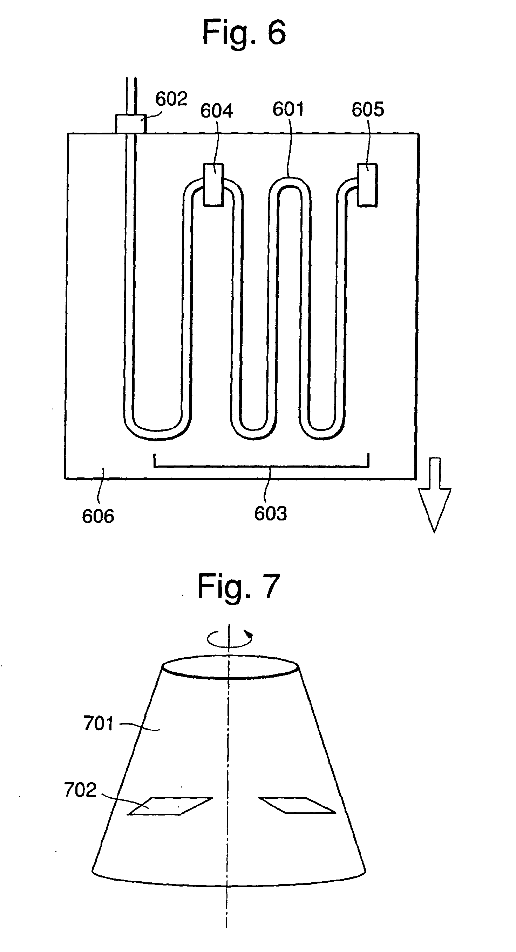

Next, description will be made of the separating means and method for separating coagulation factors from blood plasma, which is obtained from blood through the filtering means, in a microcapillary by the centrifuge, thereby obtaining serum, by referring to FIG. 6. One end of a U-shaped microcapillary 601 is connected to an inlet 602 having a needle. Also, an electro osmotic flow pump 603 is provided, and an electrode A (604) and an electrode B (605) are provided for applying high voltages. An electric field of about 1 kV/cm was applied between the electrodes A (604) and B (605), so that the blood is introduced in the U-shaped miccrocapillary 601 from the needle 602. In this case, the electrode A (604) was grounded so as to prevent an electric shock. A quartz plate 702 having the microcapillary of FIG. 6 was attached to a desk-top-size centrifuge 701 shown in FIG. 7, and rotated so as to apply greater acceleration in an arrow direction of FIG. 6. After 30-minute rotation at a speed of 5000 rpm, serum was obtained in a U-shaped part of the U-shaped capillary 601 opposite the arrow direction of FIG. 6.

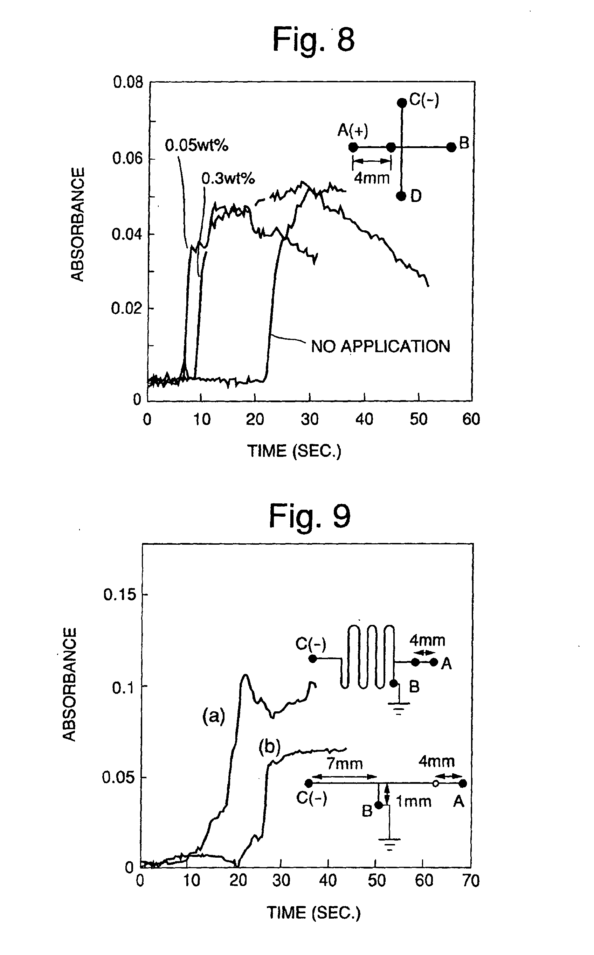

The microcapillary constituting the structure of the above-described sampling, filtering, separating, flow channel or path and analyzing means is made of quartz. Accordingly, when a biological material such as blood, blood plasma or serum is introduced into the microcapillary, protein is coagulated or adhered to an inner wall. Therefore, there is seen a phenomenon of a narrowed flow channel of the capillary, or clogging in an extreme case. In order to prevent such coagulation or adherence, MPC polymer. (2-methacryloyloxyethylphosphorylcholine) was applied onto the inner wall of the microcapillary. The MPC polymer was invented by ISHIHARA et al., (Japanese Patent No. 2,947,298), which was artificially synthesized from a material similar to that constituting biomembrane of living organisms including human. Thus, the MPC polymer is highly advantageous for preventing protein adsorption and undesirable reaction with the living organism. Now, by using a microcapillary shown in a graph of FIG. 8, description is made of presence of MPC polymer on an inner wall of the microcapillary, and an advantage of concentration of the MPC polymer applied onto the inner wall for preventing coagulation or adherence of protein or the like. The microcapillary has a width of about 30 µm, and a depth of about 30 µm. First, the microcapillary was filled with a phosphoric buffer solution of pH 7.4 and ionic strength 0.16. After bovine serum was injected to an capillary end A, various high electric fields were applied between A and C, and the serum was introduced into the capillary. For an effect of coagulation or adherence on a flow of serum in the microcapillary, concentration of the introduced serum was measured by irradiating the serum with ultraviolet ray having peaked wavelength of 220 nm, which is strongly absorbed by the serum, at a point E away from the capillary end A by 4 mm, and determining by the UV absorption whether the serum reaches to the point E. The measurement was carried out for each of no application of MPC polymer as well as applications of MPC polymmer at 0.05 wt% concentration and 0.3 wt% concentration. In the case of the MPC polymer application, not many negative charges are generated on an MPC polymer surface compared with a quartz surface. It is accordingly expected that the MPC polymer application suppresses the electro osmotic flow and that the serum in the case of no MPC polymer application reaches fastest to the measuring point. However, a result of the measurement showed that serum in the case of no application reached last the measuring point. There is not much difference in reaching time between 0.05 wt% and 0.3 wt%. However, in the case of 0.3 wt%, the serum concentration is saturated after reaching, while clear reductions occur in the cases of 0.05 wt% and no application. Thus, in the cases of no application and 0.05 wt%, MPC application was insufficient, and the proteins in the serum were conceivably adhered to the inner wall and lost before reaching the measuring point. In other words, the application of the MPC polymer of 0.3 wt% concentration on the surface of the quartz microcapillary proved to be advantageous for suppressing coagulation or adherence in measurement of serum protein in the microcapillary.

Next, description will be made of problems and an example of countermeasures especially when an ion sensitive field effect transistor (ISFET) is used as analyzing means in measurement of serum by a chemical sensor. The ISFET has a MOS (metal-oxide-semiconductor) structure. As shown in the graph of FIG. 8, serum is introduced from the microcapillary end A, and a high voltage is applied between A and C. When a measuring area of the ISFET is set between the A and C in order to measure ionic concentration in the serum moving between the A and C at, for example a point E, a high voltage is directly applied to the MOS structure, causing a dielectric breakdown in an insulating film of the ISFET laminating nitride silicon film and oxide silicon film even if an FET is set in a floating state. As a countermeasure, an electro osmotic flow pump was provided in a downstream side of the microcapillary to apply a high voltage. By a sucking function of the pump, serum was introduced to the microcapillary positioned more upstream than the pump. A chemical sensor such as an ISFET was provided in this portion of the microcapillary. FIG. 9 shows comparison of serum moving states by an operation of an electro osmotic flow between a case (a) where a microcapillary pump area having a length of 11 cm is provided in a downstream side, and a case (b) where a very short pump area of 0.8 cm is provided. The microcapillary has a width of about 30 µm, and a depth of about 30 µm. First, a microcapillary flow path was filled throughout with PBS and, in each microcapillary, a voltage of 2 kV was applied between B and C. Then, bovine serum was introduced from the inlet A, and irradiated with ultraviolet ray having peaked wavlength of 220 nm at the point E. A change of absorbance with time in this case is shown. As a result, with voltages set equal, the serum reached a position of 4 mm from the inlet A faster in the capillary form of the case (a) than that in the case (b). A high pumping capability was obtained by making longer the microcapillary between the B and C. Thus, even in the case where the moving means of serum or the like was provided in the downstream side of the chemical sensor such as an ISFET, the serum was guided to the chemical sensor, exhibiting capability of preventing dielectric breakdown of the ISFET.

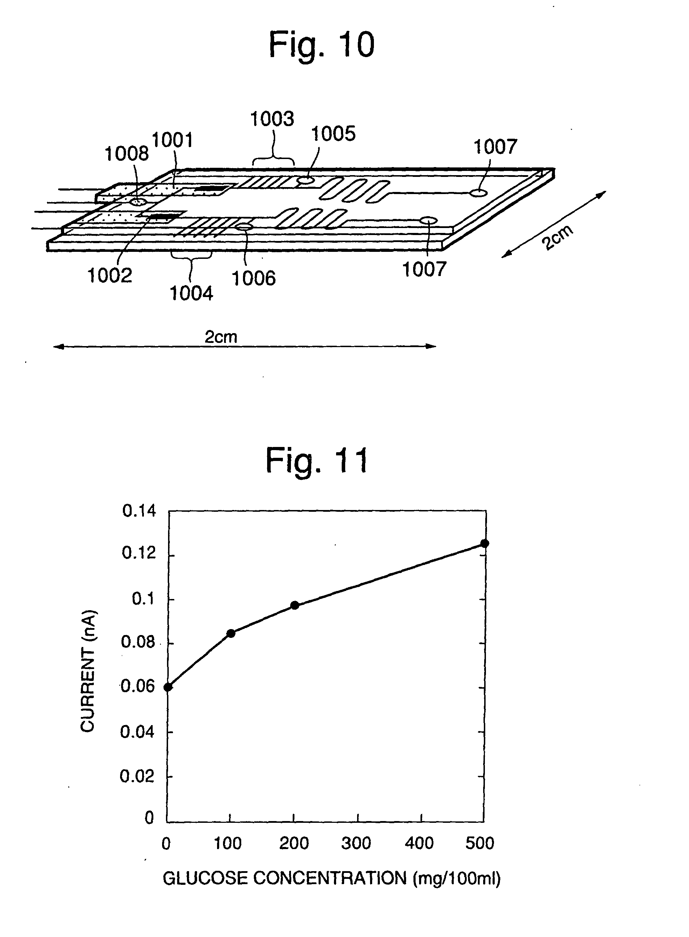

Next, description will be made of blood analysis conducted by using the present apparatus, and its result. FIG. 10 shows a configuration in the vicinity of the analyzing means of the apparatus. Reference numerals 1001 and 1002 denote ISFET sensors, 1003 and 1004 a glucose sensor and an Ag/AgCl electrode, and 1005 an electro osmotic flow pump as moving means. The ISFET has a measuring area of 15 µm x 150 µm. It was arranged along a flow path of a microcapillary having a width 30 µm and below the same. A platinum wire glucose sensor having a platinum diameter of 20 µm was inserted vertically to a side wall of the microcapillary flow path.

FIG. 11 shows a sensor current with respect to a change of concentration in a case where glucoses different in concentration are mixed in bovine serum. Here, as a glucose sensor, one constructed by sequentially applying cellulose acetate, glucose oxidase, and ferrocene carboxyaldehyde onto a Pt wire having a diameter of 20 µm was used. Surfaces thereof were coated with MPC polymer. FIG. 11 shows that substantially linear response is made to a change of glucose concentration, while a current flowing at 0 concentration of glucose is a dark current.

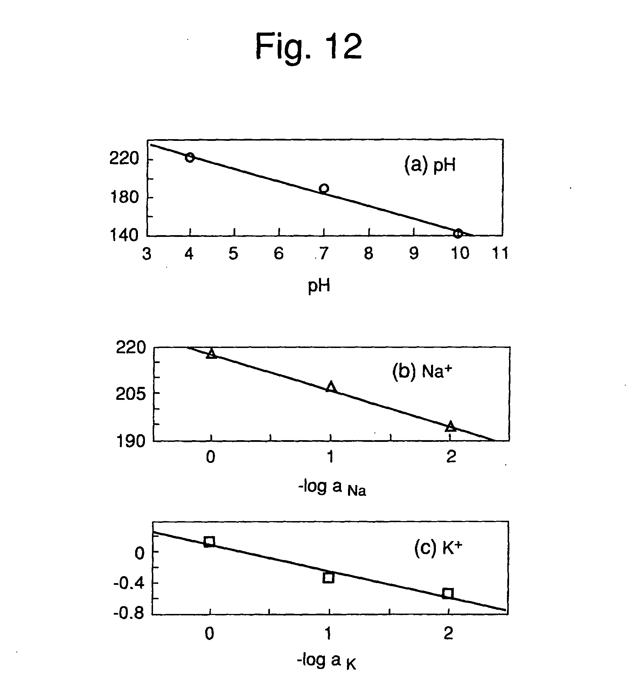

FIG. 12 shows a result of measuring pH and concentration of each of Na⁺ and K⁺ in the bovine serum using the ISFET sensors. For measurements of pH, Na⁺ and K⁺, an Si₃N₄ film; a film of a mixture of PVC, THF, BIS(12-crown-4), NPOE and K-TCPG; and a mixed film of PVC, THF, BIS(Benzo-15-crown-5), NPOE and K-TCPG were respectively used as sensitive film. In all of these cases, responses were linear, and wide-ranging.

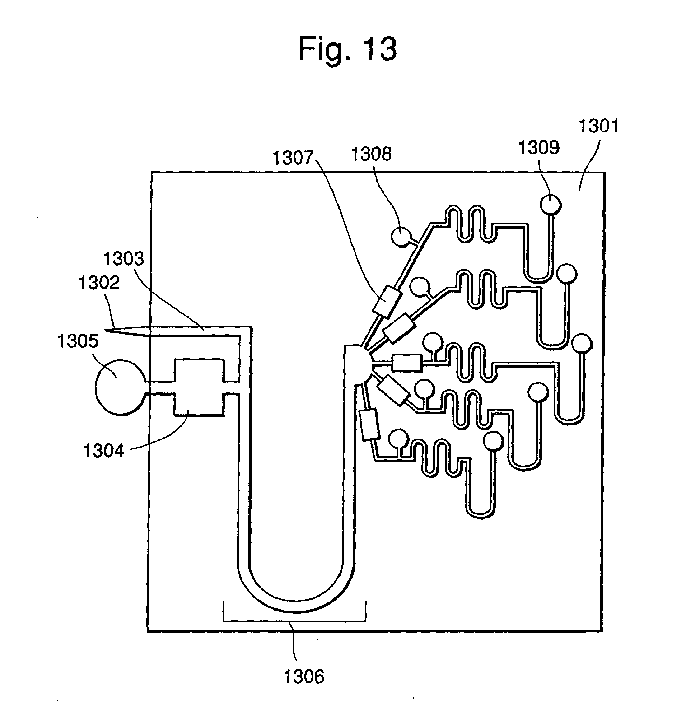

FIG. 13 shows a configuration of the blood analyzing apparatus of the invention described above with reference to FIG. 2, to which a blood anticoagulant feeder is attached. In the apparatus, as similar to the example of FIG. 2, first, blood is introduced to a microcapillary 1303 on a quartz substrate 1301 through a needle 1302 for sampling blood. In this case, anticoagulant (sodium citrate, EDTA, or heparin) stored in a portion 1304 to prevent coagulation of blood in a capillary can be properly fed into the capillary by pressing a rubber stopper 1305. Then, by using the centrifuge shown in FIG. 7, serum and blood cells are separated at separating means 1306. The serum is then guided to analyzing means 1307 by an electro osmotic flow generated by applying an electric field between electrodes 1308, 1309, and pH and concentrations of sodium ions, potassium ions, glucose and the like in the serum are detected. In the embodiment, a plurality of such analyzing means are installed, enabling concentrations of such components to be analyzed all at once. In actual investigation of these concentrations in serum, they were measured with accuracy equal to that of the foregoing measuring result.

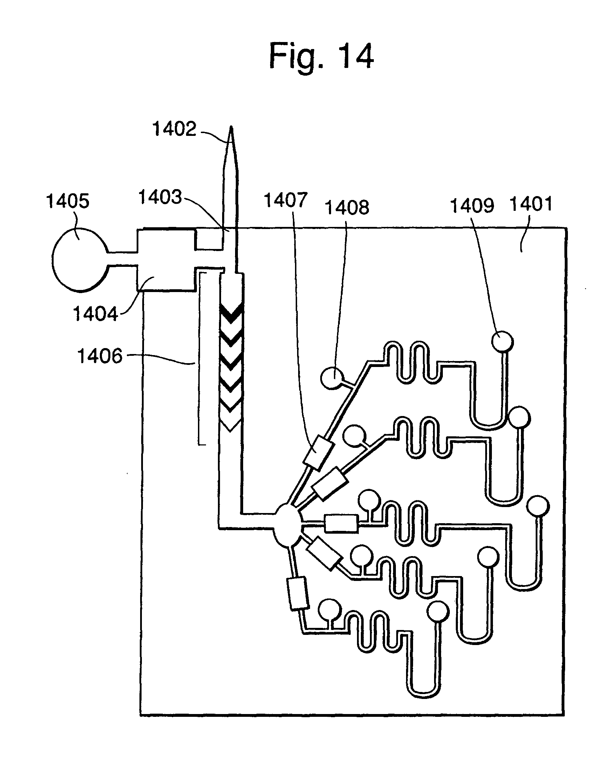

FIG. 14 shows a configuration of the blood analyzing apparatus of the invention described above with reference to FIG. 2, to which a blood anticoagulant feeder is attached. In the apparatus, as similar to the example of FIG. 2, first, blood is introduced to a microcapillary 1403 on a quartz substrate 1401 through a needle 1402 for sampling blood. In this case, anticoagulant (sodium citrate, EDTA, or heparin) stored in a portion 1404 to prevent coagulation of blood in a capillary may be properly fed into the capillary by pressing a rubber stopper 1405. Then, blood plasma and blood cells are separated by filtering means 1406. The blood plasma is then guided to analyzing means 1407 by an electro osmotic flow generated by applying an electric field between electrodes 1408, 1409, and pH and concentrations of sodium ions, potassium ions, glucose and the like in the blood plasma are detected. In the embodiment, a plurality of such analyzing means are installed, enabling concentrations of such components to be analyzed all at once. In actual investigation of these concentrations in blood plasma filtered by the filtering means 1406, they were measured with accuracy equal to that of the foregoing measuring result.

### Industrial Applicability

As apparent from the foregoing, the blood analyzing apparatus of the present invention realizes compactness, and is suitable for installation at ordinary home. Moreover, the blood analyzing method of the invention conducts procedures from blood sampling to analyzing in an integrated process. Thus, it is suitable for use by an ordinary person having no special medical knowledge or qualification.

## Claims

1. A method of sampling, filtering, separating and analyzing blood in an apparatus, said apparatus comprising, in one or a plurality of substrates;
sampling means for sampling blood from a living body;
at least one of filtering means and separating means, the filtering means being for filtering at least the sampled blood to obtain blood plasma, the separating means being for separating serum from the blood;
analyzing means for analyzing a substance in the blood;
flow path means for interconnecting the sampling means, the filtering means, the separating means and the analyzing means;
moving means for moving components of the blood present in the sampling means, the filtering means, the separating means, the analyzing means and the flow path means;
outputting means for taking out information from the analyzing means; and
control means for controlling an operation of at least one of the sampling means, the filtering means, the separating means, the analyzing means, the moving means and the outputting means;
wherein said substrate further comprises holding means for holding the blood components in the substrate, and if the plurality of the substrates are present, these substrates are integrally constituted.

2. A blood analyzing apparatus comprising, in one or a plurality of substrates:
sampling means for sampling blood from a living body;
at least one of filtering means and separating means, the filtering means being for filtering at least the sampled blood to obtain blood plasma, the separating means being for separating serum from the blood; analyzing means for analyzing a substance in the blood;
flow path means for interconnecting the sampling means, the filtering means, the separating means and the analyzing means;
moving means for moving components of the blood present in the sampling means, the filtering means, the separating means, the analyzing means and the flow path means;
outputting means for taking out information from the analyzing means; and
control means for controlling an operation of at least one of the sampling means, the filtering means, the separating means, the analyzing means, the moving means and the outputting means;
wherein the substrate further comprises holding means for holding the blood components in the substrate, and if the plurality of the substrates are present, these substrates are integrally constituted.

3. The blood analyzing method according to claim 1,
wherein prior to the blood sampling, the sampling means, the filtering means, the separating means, the analyzing means, the moving means and the flow path means are filled with a buffer solution.

4. A method comprising; rotating a main body of the blood analyzing apparatus of claim 2 together with blood or a blood extract present in the separating means of claim 2, so that the blood components are separated in the separating means due to a difference in centrifugal forces which act on the blood components having different specific gravities in the blood.

5. The blood analyzing apparatus according to claim 2, wherein the moving means for introducing the blood components to the analyzing means is installed on a downstream side of the analyzing means.

6. The blood analyzing apparatus according to claim 2, wherein, of two electrodes constituting the moving means of claim 2, one electrode which is nearer to the sampling means along a flow path is grounded.

7. The blood analyzing apparatus according to claim 2, wherein the analyzing means is means for measuring a pH value as well as concentrations of oxygen, carbon dioxide, sodium, potassium, calcium, glucose and lactic acid in the serum.

8. An apparatus in which:
MPC polymer (2-methacryloyloxyethylphosphorylcholine) having a concentration of from 0.25 wt% to 1.0 wt% is applied onto at least an inner wall of a microcapillary, which is constituted of a groove-shaped structure having a width of from 10 µm to 150 µm and a depth of from 10 µm and 150 µm, and a cap for holding a material including at least a liquid in a groove of the groove-shaped structure.
